# EUROPEAN PATENT SPECIFICATION

(11) **EP 4 321 502 B1**
(45) Date of publication and mention of the grant of the patent: **03.06.2026**
(21) Application number: 22783971.9
(22) Date of filing: 01.04.2022
(51) Int. Cl.: C07C 67/03, C07C 69/732, C09K 15/08, C08K 5/134, C08K 5/372

(54) **INTRAMOLECULAR COMPLEX HINDERED PHENOL COMPOUND, PREPARATION METHOD THEREFOR AND USE THEREOF**
INTRAMOLEKULARE KOMPLEXGEHINDERTE PHENOLVERBINDUNG, HERSTELLUNGSVERFAHREN DAFÜR UND VERWENDUNG DAVON
COMPOSÉ PHÉNOLIQUE À ENCOMBREMENT STÉRIQUE PAR UN COMPLEXE INTRAMOLÉCULAIRE, SON PROCÉDÉ DE PRÉPARATION ET SON UTILISATION

(30) Priority: 08.04.2021 CN 202110377984
(43) Date of publication of application: 14.02.2024
(73) Proprietor: Rianlon Corporation, Tianjin 300480 (CN)
(72) Inventor: SUN, Chunguang, Tianjin 300480 (CN); LIN, Xufeng, Tianjin 300480 (CN); FAN, Xiaopeng, Tianjin 300480 (CN); LI, Haiping, Tianjin 300480 (CN)
(74) Representative: Latscha Schöllhorn Partner AG
(86) International application number: PCT/CN2022/084886
(87) International publication number: WO 2022/213904

(56) References cited:
- CN-A- 101 508 644
- CN-A- 106 674 002
- JP-A- S5 925 349
- US-A- 4 618 700
- US-A- 5 892 097
- US-A- 5 892 097

## Description

### Technical Field

The present invention relates to the field of polymer material auxiliaries, in particular to an intramolecular complex hindered phenolic compound, a preparation method and application thereof.

### Background Art

During the manufacturing, storage, processing, and use of polymer materials, oxidation can cause a decrease or loss in the performance of the polymer materials such as plastics and their products, leading to the aging of polymer materials. Adding antioxidants can endow polymer materials with necessary antioxidant capacity.

Since the world's first antioxidant BHT with a hindered phenolic structure was introduced in 1937, the development and research of hindered phenolic antioxidants have been attracting attention. At present, the majority of hindered phenolic antioxidants in the world are synthesized based on 2,6-di-tert-butylphenol (symmetrical type) or 2-methyl-6-tert-butylphenol (unsymmetrical type ), and the corresponding hindered phenolic antioxidants are always synthesized from a single hindered phenol. The starting hindered phenol raw material of the symmetrical hindered phenol antioxidant 1010 is only 2,6-di-tert-butylphenol (symmetrical type). Antioxidant 1010, with a chemical name of pentaerythritol tetra[β-(3,5-di-tert-butyl-4-hydroxyphenyl)propionate], is a white crystalline powder having a melting point of 110-125°C and stable chemical properties, and can be widely used in general plastics, engineering plastics, synthetic rubber, fibers, hot melt adhesives, resins, oil products, inks, coatings and other industries. However, despite its low selling price and outstanding comprehensive performance, shortcomings such as poor yellowing resistance, insufficient antioxidation efficiency, and unsatisfactory compatibility with the matrix are found in practical applications. Unsymmetrical hindered phenolic antioxidants 1790 and 245, with only 2-methyl-6-tert-butylphenol (unsymmetrical type) as the starting hindered phenol raw material, have reduced hindrance on one side of the molecular structure, which significantly increases the antioxidation efficiency. Especially hydrogen bonding association exists between the antioxidant and auxiliary antioxidants such as thioesters and the like, the synergistic effect is more significant; meanwhile, antioxidants themselves are resistant to yellowing, exhibiting excellent thermal oxidation stability and color change resistance. However, the above-mentioned unsymmetrical hindered phenolic antioxidants 1790 and 245 lack the application universality as symmetrical hindered phenolic antioxidants 1010.

In summary, hindered phenolic antioxidants in existing technologies generally have problems such as poor yellowing resistance, poor antioxidation efficiency, and poor coloring resistance to NOx.

US 5 892 097 A discloses Antioxidant 1010 and its use as a polymer antioxidant.

### Summary of Invention

The main purpose of the present invention is to provide an intramolecular complex hindered phenolic compound, a preparation method and application thereof, in order to solve the common problems, such as poor yellowing resistance, lowantioxidation efficiency, and poor coloring resistance to NOx of the hindered phenolic antioxidants in the prior art.

In order to achieve the above purpose, according to one aspect of the present invention, an intramolecular complex hindered phenolic compound is provided, which has a structure represented by formula I: 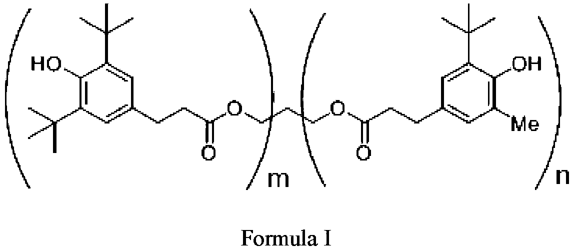 wherein, m is 1-3, n is 1-3, and m+n is 4.

Furthermore, m is 1 and n is 3; or, m is 2 and n is 2; or, m is 3 and n is 1; and preferably, m is 2 and n is 2.

According to another aspect of the present invention, a method for preparing an intramolecular complex hindered phenolic compound is also provided, which comprises the following steps: a first transesterification reaction is conducted between pentaerythritol and a first monomer to obtain an intermediate substitution product; a second transesterification reaction is conducted between the intermediate substitution product and a second monomer to obtain the intramolecular complex hindered phenolic compound; or, a third transesterification reaction is conducted between pentaerythritol and a monomer mixture to obtain the intramolecular complex hindered phenolic compound; the monomer mixture includes the first monomer and the second monomer; wherein, the first monomer and the second monomer are different, and are respectively selected from methyl 3-methyl-5-tert-butyl-4-hydroxyphenylpropionate or methyl 3,5-di-tert-butyl-4-hydroxyphenylpropionate; the substitution number of the first monomer in the intermediate substitution product corresponds to m or n in formula I; and the molar ratio of the first monomer to the second monomer in the monomer mixture corresponds to a ratio of m to n in Formula I.

Furthermore, the steps in the first, second, and third transesterification reaction are all carried out under the action of a transesterification catalyst; preferably, the transesterification catalyst is selected from one or more of alkyltin oxide, lithium amide, sodium methoxide, lithium methoxide, alkoxyaluminum, and zinc salt of organic acid; more preferably, the alkyltin oxide catalyst is selected from one or more of monobutyltin oxide and dioctyltin oxide.

Furthermore, the first monomer is methyl 3-methyl-5-tert-butyl-4-hydroxyphenylpropionate, and the second monomer is methyl 3,5-di-tert-butyl-4-hydroxyphenylpropionate; preferably, the preparation method comprises the following steps: pentaerythritol, the first monomer, and the transesterification catalyst are mixed under an inert gas, and subjected to the first transesterification reaction to obtain a pre-reaction system comprising the intermediate substitution product; the second monomer is added to the pre-reaction system and subjected to the second transesterification reaction to obtain the intramolecular complex hindered phenolic compound.

According to another aspect of the present invention, a polymer material antioxidant comprising one or more of the intramolecular complex hindered phenolic compounds mentioned above is also provided.

Furthermore, the polymer material antioxidants also include phosphite ester antioxidants and/or thioester antioxidants; preferably, the phosphite ester antioxidant is one or more of tris(2,4-di-tert-butylphenyl) phosphite, bis(2,4-di-tert-butylphenyl)pentaerythritol diphosphite, bis(2,4-dicumylphenyl)pentaerythritol diphosphite, triisodecyl phosphite, diisodecyl pentaerythritol diphosphite, tetraphenyl dipropylene glycol diphosphite, diphenyl monoisodecyl phosphite, triphenyl phosphite, monophenyl diisodecyl phosphite, tris(nonylphenyl) phosphite; preferably, the thioester antioxidant is one or more of pentaerythritol tetra(3-decylthiopropionate), dioctadecanol thiodipropionate, didodecanol thiodipropionate, ditetradecanol thiodipropionate, and ditridecanol thiodipropionate.

According to another aspect of the present invention, there is also provided a polymer material comprising a body of the polymer material and an antioxidant, wherein the antioxidant is the aforementioned polymer material antioxidant.

Furthermore, the body of the polymer material is resin or rubber; the resin is preferably polyolefin, polyurethane, polyether, polyketone, polystyrene, polycarbonate, polyacrylate, polymethacrylate, acetal, halogen-containing polymer, polyamide, polyformaldehyde, polyphenylene ether, polyethylene terephthalate, polybutylene terephthalate, polyacrylonitrile, polybutadiene, polystyrene, HIPS, ABS, MBS, epoxy resin or acrylic resin cross-linked with epoxy resin; and the rubber is preferably natural rubber, styrene butadiene rubber, chloroprene rubber, cis-1,4-polybutadiene rubber, isoprene rubber, nitrile rubber, or thermoplastic TPU.

Furthermore, the body of the polymer material is thermoplastic TPU, and the addition amount of the antioxidant is 0.01-3% by weight of the body of the polymer material; alternatively, the polymer material is PP, and the addition amount of the antioxidant is 0.01-1% by weight of the body of the polymer material; preferably, when the polymer material is PP, the antioxidant comprises the aforementioned intramolecular complex hindered phenolic compound and the antioxidant DSTDP, and more preferably, the weight ratio of the two is 1:5-5:1.

The intramolecular complex hindered phenolic compound provided by the present invention is pentaerythritol m[β-(3,5-di-tert-butyl-4-hydroxyphenyl)propionate]-n[β-(3-methyl-5-tert-butyl-4-hydroxyphenyl)propion ate, which has a good yellowing resistance, as well as advantages such as high antioxidation efficiency, good compatibility with the matrix, low melting point, and wide application range. Especially when used in the field of plastic processing, it shows resistance to NOx coloring and has good application performance.

### Description of Drawings

The accompanying drawings forming a part of the present application are used to provide further understanding of the present invention. The illustrated examples and explanations of the present invention are used to explain the present invention and do not constitute improper limitation of the present invention. In the attached drawings:
Figure 1 shows an infrared spectrum of an intramolecular complex hindered phenolic compound prepared according to Example 1 of the present invention;
Figure 2 shows a hydrogen nuclear magnetic resonance spectrum of the intramolecular complex hindered phenolic compound prepared according to Example 1 of the present invention;
Figure 3 shows a carbon nuclear magnetic spectrum of the intramolecular complex hindered phenolic compound prepared according to Example 1 of the present invention; and
Figure 4 shows a mass spectrum of the intramolecular complex hindered phenolic compound prepared according to Example 1 of the present invention.
Figure 5 shows an infrared spectrum of an intramolecular complex hindered phenolic compound prepared according to Example 3 of the present invention;
Figure 6 shows a hydrogen nuclear magnetic resonance spectrum of the intramolecular complex hindered phenolic compound prepared according to Example 3 of the present invention;
Figure 7 shows a carbon nuclear magnetic spectrum of the intramolecular complex hindered phenolic compound prepared according to Example 3 of the present invention; and
Figure 8 shows a mass spectrum of the intramolecular complex hindered phenolic compound prepared according to Example 3 of the present invention.
Figure 9 shows an infrared spectrum of the intramolecular complex hindered phenolic compound prepared according to Example 4 of the present invention;
Figure 10 shows a hydrogen nuclear magnetic resonance spectrum of the intramolecular complex hindered phenolic compound prepared according to Example 4 of the present invention;
Figure 11 shows a carbon nuclear magnetic spectrum of the intramolecular complex hindered phenolic compound prepared according to Example 4 of the present invention; and
Figure 12 shows a mass spectrum of the intramolecular complex hindered phenolic compound prepared according to Example 4 of the present invention.

### DETAILED DESCRIPTION

It should be noted that in the absence of conflicts, the examples and the features in the examples in this application can be combined with each other. The present invention will be explained in detail below with reference to the accompanying drawings and examples.

As described in the background art, the hindered phenolic antioxidants in existing technologies generally have problems such as poor yellowing resistance, low antioxidation efficiency, and poor color resistance to NOx.

To address the above issues, the present invention provides an intramolecular complex hindered phenolic compound having a structure represented by formula I: 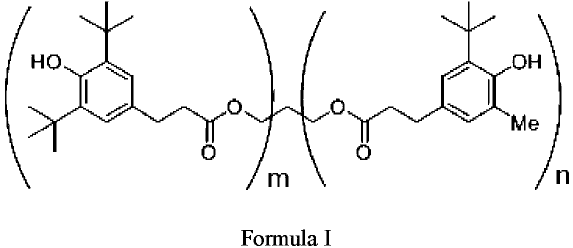 wherein, m is 1-3, n is 1-3, and m+n is 4.

The intramolecular complex hindered phenolic compound provided by the present invention is pentaerythritol m[β-(3,5-di-tert-butyl-4-hydroxyphenyl)propionate]-n[β-(3-methyl-5-tert-butyl-4-hydroxyphenyl)propion ate], which has a good yellowing resistance characteristic, as well as advantages such as high antioxidation efficiency, good compatibility with the matrix, low melting point, and wide application range. Especially used in the field of plastic processing, it shows resistance to NOx coloring and has good application performance.

Unlike traditional hindered phenols, the hindered phenolic compound of the present invention forms a new symmetrical-unsymmetrical complex structure within a molecule. In the molecular structure of symmetrical hindered phenolic antioxidant, there are two tert butyls at the ortho positions of the phenol hydroxyl group, which increases the electron cloud density of the phenoxy radical conjugated system, endows it with good stability and exhibits high antioxidation efficiency. At the same time, the steric hindrance effect of the large tert butyl groups has a protective effect on the hydroxyl group, which can prevent the oxidation loss of the antioxidant. In the molecular structure of unsymmetrical hindered phenolic antioxidants, there are tert butyl and methyl groups respectively on each ortho position of the phenol hydroxyl group, which reduce the steric hindrance effect of the phenol hydroxyl group and make it easier to form intermolecular hydrogen bonds with other materials, and thus improve the synergistic stability effect; they also make it easier for the meta position of phenol hydroxyl group to undergo a nitro substitution reaction, forming stable meta-nitro compounds that are generally white, overcoming the drawbacks of para-nitro group of completely symmetrical hindered phenol being oxidized into easily-colored products. In other words, unsymmetrical hindered phenolic antioxidants have the ability of yellowing resistance. The above hindered phenolic compound of the present invention, as the intramolecular symmetrical-unsymmetrical hindered phenolic complex antioxidant, combines the advantages of both symmetrical and unsymmetrical hindered phenolic antioxidants. In addition, the hindered phenolic compound of the present invention has a significant performance - it can significantly reduce the melting point of the product (especially compared to the symmetrical hindered phenolic antioxidant 1010 in the prior art), making the processing process more operable and convenient for feeding.

In practical applications, one of the above-mentioned intramolecular complex hindered phenolic compounds can be used as a polymer material antioxidant alone, or at least two of them can be mixed in any proportion as the polymer material antioxidant.

In a preferable embodiment, m is 1 and n is 3, corresponding to the following compound 1; alternatively, m is 2 and n is 2, corresponding to the following compound 2; alternatively, m is 3 and n is 1, corresponding to the following compound 3.

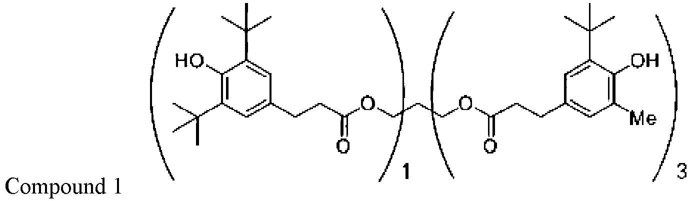

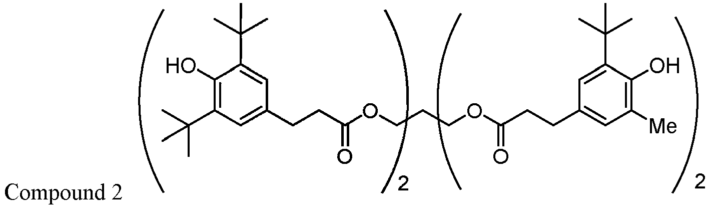

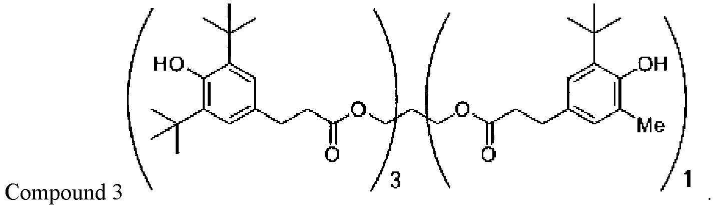

For the purpose of further improving the comprehensive performance including yellowing resistance, antioxidation efficiency, compatibility with the matrix, and NOx resistance to coloring, more preferably, m is 2, n is 2, corresponding to the above compound 2.

According to another aspect of the present invention, a method for preparing the intramolecular complex hindered phenolic compound is also provided, which includes the following steps: a first transesterification reaction is conducted between pentaerythritol and a first monomer to obtain an intermediate substitution product; a second transesterification reaction is conducted between the intermediate substitution product and a second monomer to obtain the intramolecular complex hindered phenolic compound; alternatively, a third transesterification reaction is conducted between pentaerythritol and a monomer mixture to obtain the intramolecular complex hindered phenolic compound; the monomer mixture includes the first monomer and the second monomer; wherein, the first monomer and the second monomer are different, and are respectively selected from methyl 3-methyl-5-tert-butyl-4-hydroxyphenylpropionate or methyl 3,5-di-tert-butyl-4-hydroxyphenylpropionate; the substitution number of the first monomer in the intermediate substitution product corresponds to m or n in formula I; and the molar ratio of the first monomer to the second monomer in the monomer mixture corresponds to a ratio of m to n in Formula I.

In one method: firstly an transesterification reaction between pentaerythritol and methyl 3-methyl-5-tert-butyl-4-hydroxyphenylpropionate (or methyl 3,5-di-tert-butyl-4-hydroxyphenylpropionate) is performed, causing 1 to 3 hydroxyl groups in pentaerythritol (depending on the first monomer and the values of m or n) to react to generate an intermediate substitution product; and then methyl 3,5-di-tert-butyl-4-hydroxyphenylpropionate (or methyl 3-methyl-5-tert-butyl-4-hydroxyphenylpropionate) is added for a further transesterification reaction, wherein the remaining hydroxyl groups of pentaerythritol are replaced to form the target product, i.e., the intramolecular complex hindered phenolic compound. In another method: mixed monomers of pentaerythritol, methyl 3-methyl-5-tert-butyl-4-hydroxyphenylpropionate, and methyl 3,5-di-tert-butyl-4-hydroxyphenylpropionate in the proportion of target m and n are prepared during the compounding stage of the raw material; and the mixed monomers are directly used for transesterification reaction with pentaerythritol to form the target product, i.e., the complex hindered phenolic compound within in one step.

The above manners of conducting two-step consecutive transesterification reactions or using mixed monomers for one-step transesterification reaction, in accordance with the proportion of reaction materials, can control the m and n values in the finished intramolecular complex hindered phenolic compound. However, it is inevitable that there are trace production components having substitution numbers of two monomers not matching the target m and n values, which should be understood by technical personnel in this field. The product in the form of a mixture can be purified subsequently through separation, and even if not purified, the form of a mixture can be used as a polymer antioxidant without affecting its effectiveness.

Through the method of the present invention, the steps are simple, the reaction efficiency is high, and the synthesis is convenient. The intramolecular complex hindered phenolic compound prepared is pentaerythritol m[β-(3,5-di-tert-butyl-4-hydroxyphenyl)propionate]-n[β-(3-methyl-5-tert-butyl-4-hydroxyphenyl)propion ate], which has a good yellowing resistance, as well as advantages such as high antioxidation efficiency, good compatibility with the matrix, low melting point, and wide application range. Especially used in the field of plastic processing, it shows resistance to NOx coloring and has good application performance.

In order to further improve the efficiency of the transesterification reaction, in a preferable embodiment, the first transesterification reaction step, the second transesterification reaction step, and the third transesterification reaction step are all carried out under the action of the transesterification catalyst. Preferably, the transesterification catalyst is selected from one or more of alkyltin oxide, lithium amide, sodium methoxide, lithium methoxide, alkoxyaluminum, and zinc salt of organic acid. The use of the above types of transesterification catalysts not only brings high catalytic activity and can further improve reaction efficiency, but also has milder reaction conditions. More preferably, the alkyltin oxide catalyst is selected from one or more of monobutyl tin oxide and dioctyl tin oxide.

In a preferable embodiment, the first monomer mentioned above is methyl 3-methyl-5-tert-butyl-4-hydroxyphenylpropionate, and the second monomer is methyl 3,5-di-tert-butyl-4-hydroxyphenylpropionate. In this way, the reaction scheme can proceed as follows:

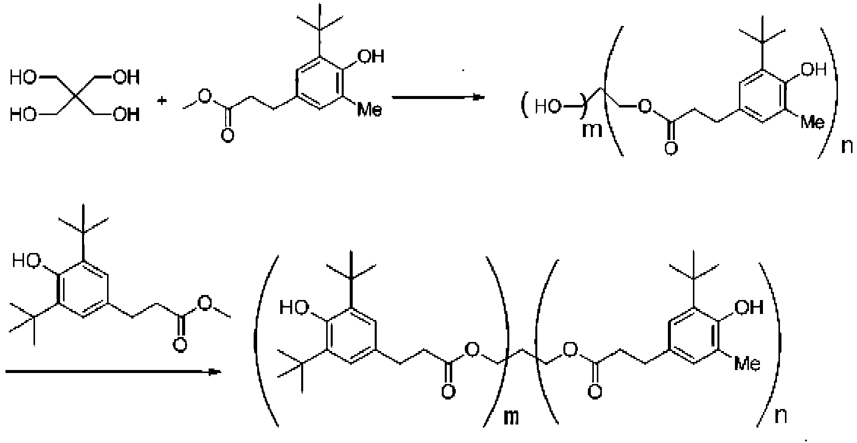

In order to further improve safety and stability in reaction, in a preferable embodiment, the preparation method comprises the following steps: pentaerythritol, the first monomer, and the transesterification catalyst are mixed under an inert gas, and the first transesterification reaction is conducted to obtain the pre-reaction system comprising the intermediate substitution product; the second monomer is added to the pre-reaction system for the second transesterification reaction to obtain the intramolecular complex hindered phenolic compound.

More preferably, the first transesterification reaction specifically includes the following steps: pentaerythritol, the first monomer, and the transesterification catalyst are mixed under an inert gas, and then heated to 120-180°C for insulation reaction; the pre-reaction system is purged with an inert gas to remove methanol; continuing to heat up to 180-185°C for insulation reaction to obtain the pre-reaction system. Preferably, the second transesterification reaction comprises the following steps: cooling the pre-reaction system to 80-150°C, then adding the second monomer thereto, and heating to 180-185°C for insulation reaction with a reaction pressure of 66.661 - 101.325 kPa (500-760 mmHg) to obtain the intramolecular complex hindered phenolic compound.

Controlling the reaction temperature of each step within the above range is beneficial for further improving reaction efficiency. At the same time, reacting in the presence of an inert gas and using the inert gas to remove the by-product methanol during the reaction is beneficial for promoting the forward progress of the reaction. The inert gases can specifically be nitrogen, argon, etc.

After the two-step ester exchange reaction, a crude product of the intramolecular complex hindered phenolic compound is obtained. In the actual synthesis process, the crude product can be purified after the reaction is completed, and the following method is preferred for purification: vacuum distillation is performed at a high vacuum of 66.661 Pa (0.5 mmHg) to recover excess second monomer. Then, the reaction solution is cooled to 115°C and dissolved in toluene under a nitrogen flow atmosphere. Then, it is washed thoroughly with a 5% oxalic acid solution and washed twice with water. The toluene phase is concentrated and dried in vacuum to obtain the pure target product..

In order to further promote the forward progress of the reaction and avoid replacing excess hydroxyl groups with too many first monomers as much as possible, in a preferable embodiment, the weight ratio of the transesterification catalyst to pentaerythritol is (0.03-0.2):1; preferably, when the first monomer is methyl 3-methyl-5-tert-butyl-4-hydroxyphenylpropionate, the molar ratio of the first monomer to pentaerythritol is [n~(n+0.2)]:1, and the molar ratio of the second monomer to pentaerythritol is [m~(m+1)]:1; when the first monomer is methyl 3,5-di-tert-butyl-4-hydroxyphenylpropionate, the molar ratio of the first monomer to pentaerythritol is [m~(m+0.2)]:1, and the molar ratio of the second monomer to pentaerythritol is [n~(n+1)]:1; wherein, m and n are defined as mentioned above.

According to another aspect of the present invention, there is provided use of individual or mixed intramolecular complex hindered phenolic compounds as a polymer material antioxidant. As mentioned above, the intramolecular complex hindered phenolic compound is pentaerythritol m[β-(3,5-di-tert-butyl-4-hydroxyphenyl)propionate]-n[β-(3-methyl-5-tert-butyl-4-hydroxyphenyl)propion ate], which has a good yellowing resistance, as well as advantages such as high antioxidation efficiency, good compatibility with the matrix, low melting point, and wide application range. Especially used in the field of plastic processing, it shows resistance to NOx coloring and has good application performance. In practical applications, one of the above-mentioned intramolecular complex hindered phenolic compounds can be used as a polymer material antioxidant alone, or at least two of them can be mixed in any proportion as a polymer material antioxidant.

More preferably, in specific examples, the intramolecular complex hindered phenolic compound of the present invention is used in combination with phosphite ester antioxidants and thioester antioxidants to achieve a better antioxidation effect.

The aforementioned phosphite antioxidants refer to auxiliary antioxidants having a phosphite or diphosphite structure, such as tris(2,4-di-tert-butylphenyl) phosphite (antioxidant 168), bis(2,4-di-tert-butylphenyl) pentaerythritol diphosphite (antioxidant 626), bis(2,4-dicumylphenyl) pentaerythritol diphosphite (antioxidant 686), triisodecyl phosphite (antioxidant PL-81), pentaerythritol diisodecyl diphosphite (antioxidant PL-90), tetraphenyldipropylene glycol diphosphite (antioxidant THOP), diphenyl monoisodecyl phosphite (antioxidant DPDP), triphenyl phosphite (antioxidant TPPi), monophenyl diisodecyl phosphite (antioxidant PDDP), and tri(nonylphenyl) phosphite (antioxidant TNPP), most preferably, diphenyl monoisodecyl phosphite. However, the present invention is not limited to the aforementioned compounds.

The above-mentioned thioester antioxidants refer to auxiliary antioxidants having sulfur-ether groups, such as pentaerythritol tetra(3-decylthiopropionate) (antioxidant 412S), dioctadecanol thiodipropionate (antioxidant DSTDP), didodecanol thiodipropionate (antioxidant DLTDP), ditetradecanol thiodipropionate (antioxidant DMTDP), and ditridecanol thiodipropionate (antioxidant DTDTP). However, the present invention is not limited to the aforementioned compounds. In specific examples, the intramolecular complex hindered phenolic compound of the present invention can also be used in combination with auxiliary agents such as ultraviolet absorbers, hindered amine light stabilizers, flame retardants, nucleating agents, etc. The type of ultraviolet absorber is not particularly limited and can be a conventional type in this field, such as benzophenones, benzotriazoles, triazines, benzoate, etc.

According to another aspect of the present invention, there is also provided a polymer material comprising a body of the polymer material and an antioxidant. In practical application, the antioxidant of the present invention can be used in polymer materials, such as in resin or rubber; For example, the resin includes, but is not limited to, polyolefin, polyurethane, polyether, polyketone, polystyrene, polycarbonate, polyacrylate, polymethacrylate, polyacetal, halogen-containing polymer, polyamide, polyformaldehyde, polyphenylene ether, polyethylene terephthalate, polybutylene terephthalate, polyacrylonitrile, polybutadiene, polystyrene, HIPS, ABS, MBS, epoxy resin or acrylic resin crosslinked with epoxy resin; For example, the rubber includes, but is not limited to, natural rubber, styrene butadiene rubber, chloroprene rubber, cis-1,4-polybutadiene rubber, isoprene rubber, nitrile rubber, or thermoplastic TPU. The above antioxidants are preferably used as antioxidant additives for thermoplastic TPU, polypropylene PP and other polymer materials.

According to the types of the body of the polymer material, technicians in this field can choose the addition amount of antioxidant specifically. Preferably, the body of the polymer material is TPU, and the amount of antioxidant added is 0.01-3% of the weight of TPU. More preferably, the intramolecular complex hindered phenolic compound is used as the antioxidant for TPU, which has better compatibility with TPU and can fully play its role; alternatively, the polymer material is PP, and the amount of antioxidant added is 0.01-1% of the weight of PP. More preferably, the intramolecular complex hindered phenolic compound is compounded with the antioxidant DSTDP to form the antioxidant of PP, which has a more significant effect. The preferred weight ratio of the intramolecular complex hindered phenolic compound to the antioxidant DSTDP is 1:5-5: 1, more preferably 1:3.

The following provides a further detailed description of the present application in conjunction with specific examples, which cannot be understood as limiting the scope of protection required by the present application.

### Example 1:

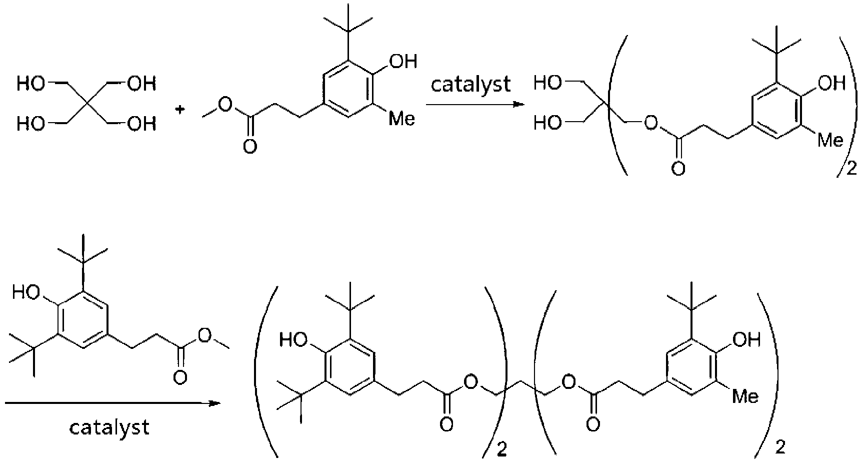

To a reaction flask 5.45 g (40 mmol) of pentaerythritol, 20 g (80 mmol) of methyl 3-methyl-5-tert-butyl-4-hydroxyphenylpropionate, and 0.7 g of dioctyltin oxide catalyst were added under a nitrogen flow atmosphere, the temperature was increased to 165°C, and the reaction was stirred and held for 3 hours. Nitrogen gas was purged to drive away the escaping methanol, and the temperature was raised to 180-185°C for 2 hours. At this point, the reaction was basically complete. Next, the temperature was lowered to 120°C, and 35.1 g (120 mmol) of 3,5-di-tert-butyl-4-hydroxyphenylpropionate methyl ester was added thereto. Under stirring and a temperature raised to 180-185°C, the reaction lasted for 8 hours. Nitrogen gas was purged to drive away the escaping methanol. Vacuum distillation was carried out at a high vacuum of 66.661 Pa (0.5 mmHg) to recover excess methyl 3,5-di-tert-butyl-4-hydroxyphenylpropionate. Then, the reaction solution was cooled to 115°C and dissolved in 400 ml of toluene added under a nitrogen flow atmosphere, and then was washed thoroughly with 200 ml of 5% oxalic acid solution and twice with water (200 ml each time). The toluene phase was concentrated and dried in vacuum to obtain 42 g of product. The main product pentaerythritol bis[β-(3,5-di-tert-butyl-4-hydroxyphenyl)propionate]-bis[β-(3-Methyl-5-tert-butyl-4-hydroxyphenyl)prop ionate (compound 2) was confirmed, the yield was 96%; and the melting point was 48~50°C. The characterization data of compound 2 are as follows:
hydrogen nuclear magnetic spectrum ¹H NMR (400 MHz, CDCl₃) δ= 6.98 (s, 4H), 6.93 (d, *J* = 1.8 Hz, 2H), 6.81 (d, *J=* 1.6 Hz, 2H), 5.08 (s, 2H), 4.68 (s, 2H), 3.99 (s, 4H), 3.97 (s, 4H), 2.85-2.79 (m, 8H), 2.60-2.56 (m, 8H), 2.19 (s, 6H), 1.43 (s, 36H), 1.39 (s, 18H).
carbon nuclear magnetic spectrum ¹³C NMR (101 MHz, CDCl₃) δ 172.66, 172.64, 152.27, 151.20, 136.07, 136.05, 131.27, 130.73, 128.13, 124.82, 124.80, 123.51, 62.34, 41.74, 36.11, 34.46, 34.44, 30.82, 30.43, 30.41, 29.80, 16.02.

High resolution mass spectrometry HRMS (EI-TOF, M+K⁺): 1131.6536.

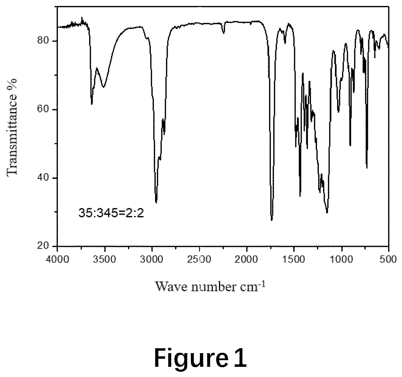

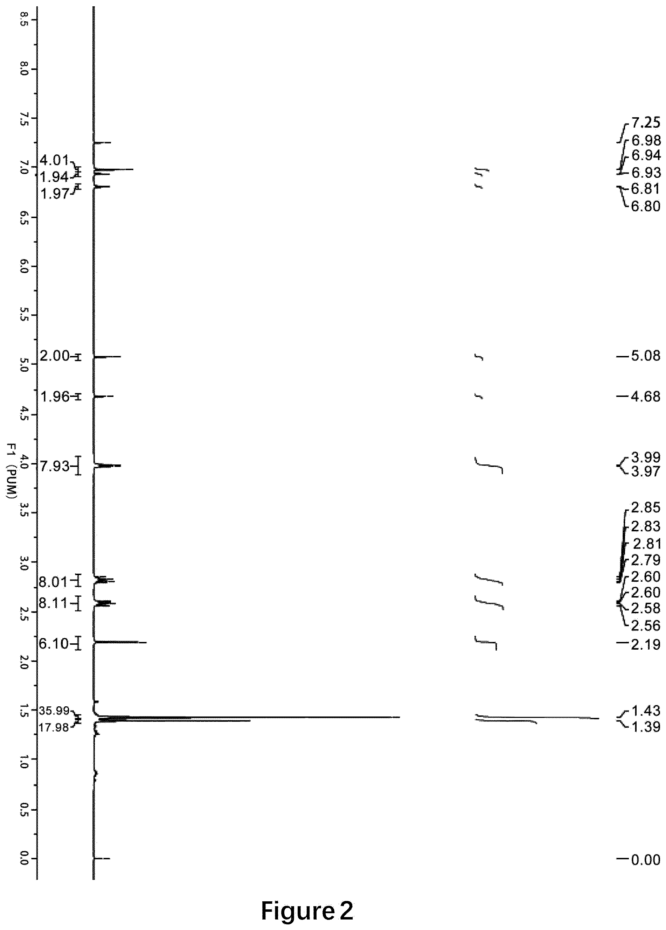

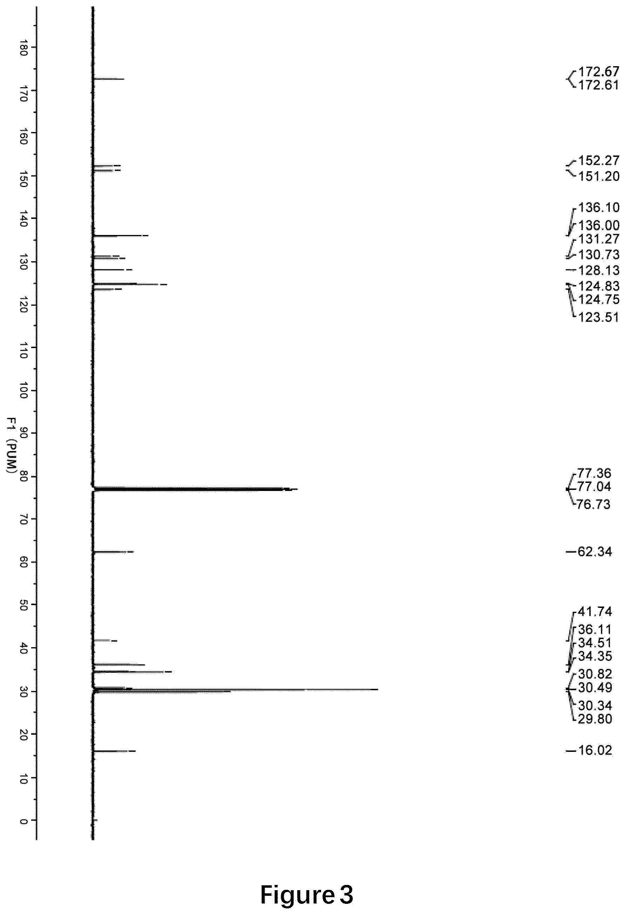

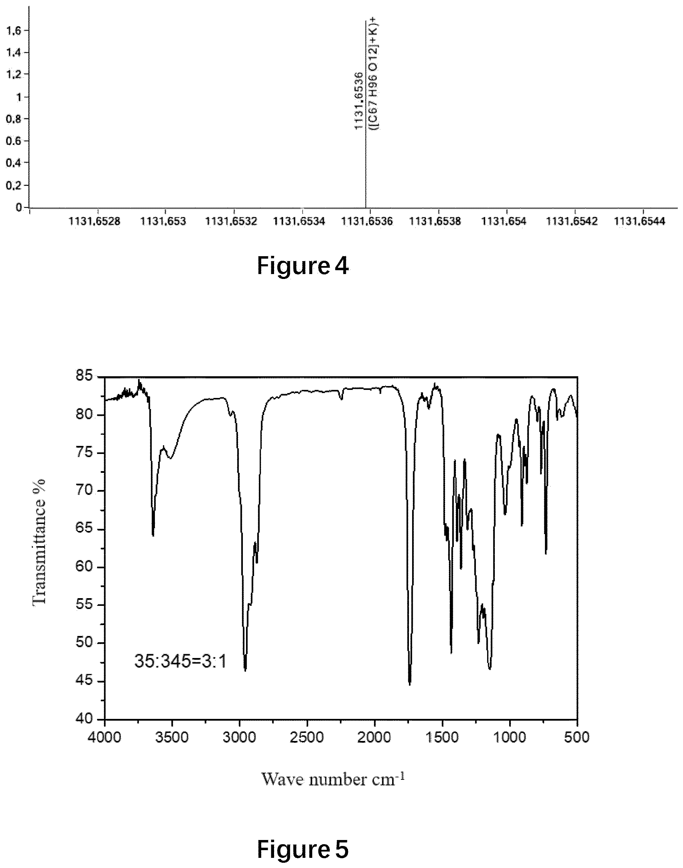

The infrared spectrum is shown in Figure 1, the hydrogen nuclear magnetic spectrum is shown in Figure 2, the carbon nuclear magnetic spectrum is shown in Figure 3, and the high-resolution mass spectrometry is shown in Figure 4.

### Example 2:

To a reaction flask 5.45 g (40 mmol) of pentaerythritol, 20 g (80 mmol) of methyl 3-methyl-5-tert-butyl-4-hydroxyphenylpropionate, and 0.2 g of monobutyl tin oxide catalyst were added under a nitrogen flow atmosphere. The temperature was raised to 165°C, and the reaction was stirred and held for 3 hours. Nitrogen gas was purged to drive away the escaping methyl alcohol, and the temperature was raised to 180-185°C for 2 hours. At this point, the reaction was basically complete. Next, the temperature was lowered to 120°C, and another 80mmol of methyl 3,5-di-tert-butyl-4-hydroxyphenylpropionate was added thereto. The vacuum was turned on, and the reaction pressure was maintain at 93.325 kPa (700mmHg). The reaction was conducted for 2 hours after the temperature was raised to 180-185°C with stirring. Then, the reaction solution was cooled to room temperature and recrystallized using a mixture of toluene and methanol to obtain 32 g of product. The main product was confirmed by hydrogen nuclear magnetic spectrum to be pentaerythritol bis[β-(3,5-di-tert-butyl-4-hydroxyphenyl)propionate]-bis[β-(3-Methyl-5-tert-butyl-4-hydroxyphenyl)prop ionate (compound 2), with a yield of 73%; a melting point 48~50°C.

### Example 3:

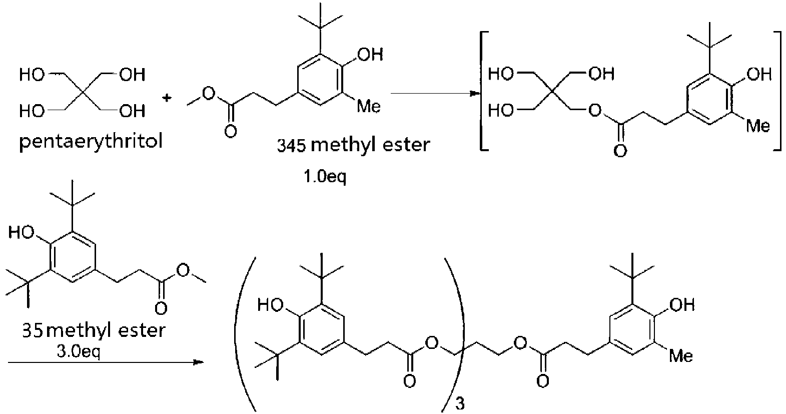

To a reaction flask 5.45 g (40mmol) of pentaerythritol, 10 g (40mmol) of methyl 3-methyl-5-tert-butyl-4-hydroxyphenylpropionate (referred to as 345 methyl ester), and 0.2 g of monobutyl tin oxide catalyst were added under a nitrogen flow atmosphere. The temperature was raised to 165°C, and the reaction was stirred and held for 3 hours. Nitrogen gas was purged to drive away the escaping methanol, and the temperature was raised to 180-185°C for 1 hour. At this point, the reaction was basically complete. Next, the temperature was lowered to 120°C, and another 120 mmol of methyl 3,5-di-tert-butyl-4-hydroxyphenylpropionate (referred to as 35 methyl ester) was added thereto. The vacuum was turned on, and the reaction pressure was maintain at 93.325 kPa (700mmHg). The reaction was conducted for 3 hours after the temperature was raised to 180-185°C. Then, the reaction solution was cooled to room temperature and recrystallized using a mixture of toluene and methanol to obtain 26 g of product. The main product was confirmed by hydrogen nuclear magnetic spectrum to be pentaerythritol tri[β-(3,5-Di-tert-butyl-4-hydroxyphenyl) propionate]-[β-(3-methyl-5-tert-butyl-4-hydroxyphenyl)propionate (compound 1), with a yield of 57%; a melting point 39-40°C,. TLC expansion agent for monitoring reaction is ethyl acetate/n-hexane=1:6; The Rf values of raw materials and various products are 0.7 for 345 methyl ester, 0.6 for 35 methyl ester, and 0.5 for compound 1. The characterization data of compound 1 are as follows:
hydrogen nuclear magnetic spectrum ¹H NMR (400 MHz, CDCl₃) δ= 6.98 (s, 6H), 6.94 (d, *J* = 1.8 Hz, 1H), 6.81 (d, *J =* 1.6 Hz, 1H), 5.08 (s, 3H), 4.67 (s, 1H), 4.03 (d, *J* = 6.9 Hz, 8H), 2.83 (dd, *J =* 15.7, 7.2 Hz, 8H), 2.60 (dd, *J =* 10.3, 6.0 Hz, 8H), 2.19 (s, 3H), 1.43 (s, 54H), 1.39 (s, 9H).
carbon nuclear magnetic spectrum ¹³C NMR (101 MHz, CDCl₃) δ. 172.67, 152.27, 151.19, 136.05, 136.06, 131.27, 130.73, 128.13, 124.80, 124.81, 123.51, 62.32, 41.83, 36.14, 34.44, 34.45, 30.82, 30.42, 30.83, 29.76, 29.77, 16.01.

High resolution mass spectrometry HRMS (EI-TOF, M+K⁺): 1173.7004.

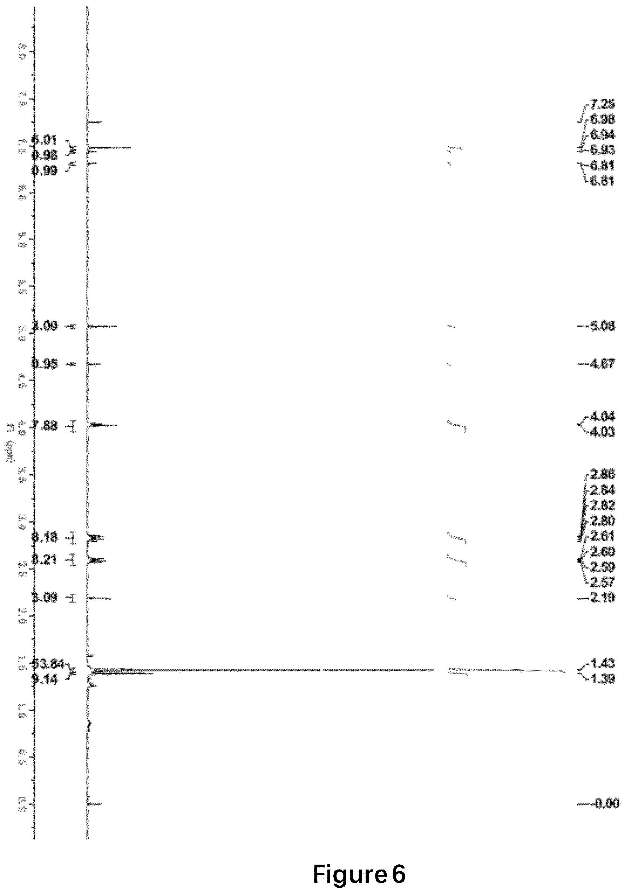

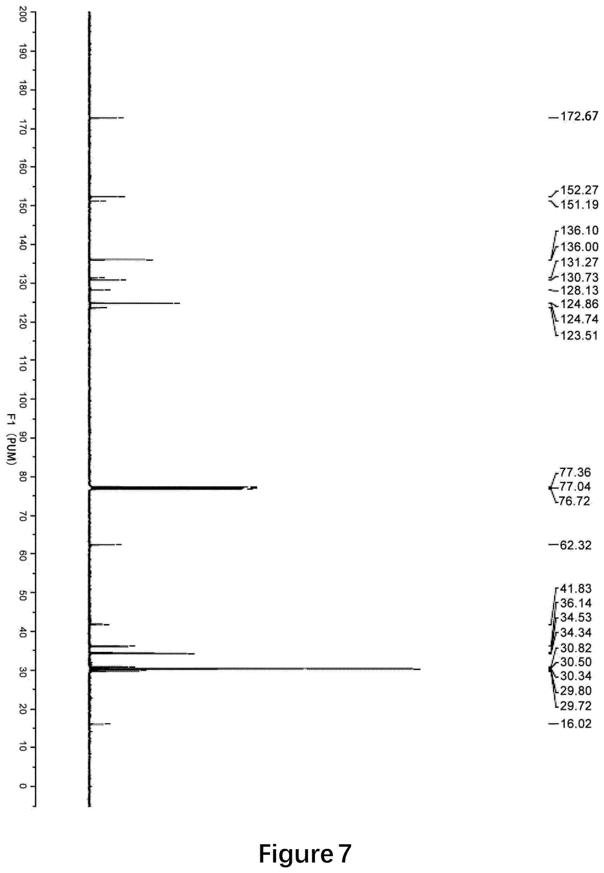

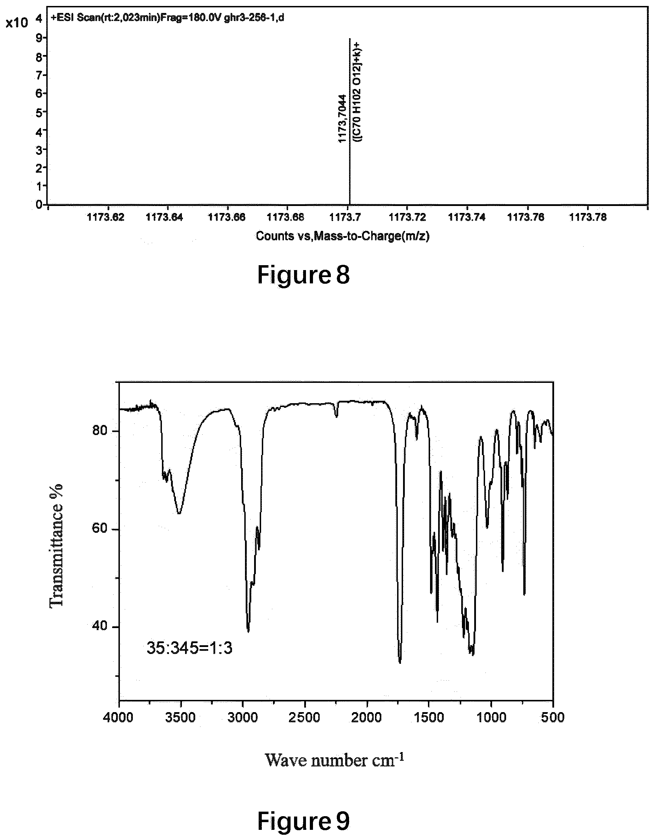

The infrared spectrum is shown in Figure 5, the hydrogen nuclear magnetic spectrum is shown in Figure 6, the carbon nuclear magnetic spectrum is shown in Figure 7, and the high-resolution mass spectrometry is shown in Figure 8.

### Example 4:

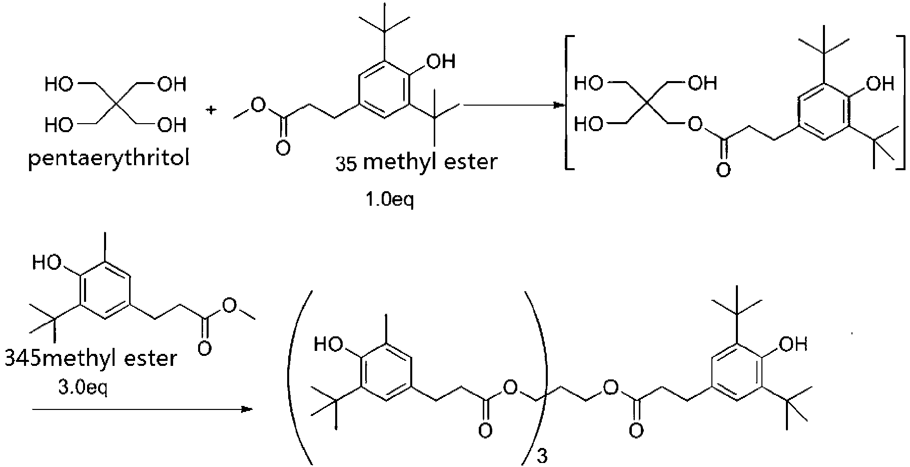

To a reaction flask 5.45 g (40mmol) of pentaerythritol, 40mmol of methyl 3,5-di-tert-butyl-4-hydroxyphenylpropionate (referred to as 35 methyl ester), and 0.2 g of monobutyl tin oxide catalyst were added under a nitrogen flow atmosphere. The temperature was raised to 165°C, and the reaction was stirred and held for 2 hours. Nitrogen gas was purged to drive away the escaping methyl alcohol, and the temperature was raised to 180-185°C for 1 hour. At this point, the reaction was basically complete. Next, the temperature was lowered to 120°C, another 30g (120mmol) of methyl 3-methyl-5-tert-butyl-4-hydroxyphenylpropionate (referred to as 345 methyl ester) was added. The vacuum was turned on, and the reaction pressure was maintain at 93.325 kPa (700mmHg). The reaction was conducted for 3 hours after the temperature was raised to 180-185°C. Then, the reaction solution was cooled to room temperature and recrystallized using a mixture of toluene and methanol to obtain 25 g of product. The main product was confirmed by hydrogen nuclear magnetic spectrum to be pentaerythritol [β-(3,5-di-tert-butyl-4-hydroxyphenyl)propionate]-tris[β-(3-methyl-5-tert-butyl-4-hydroxyphenyl)propion ate (compound 3), with a melting point 49-50°C and a product yield of 60%. TLC expansion agent for monitoring reaction is ethyl acetate/n-hexane=1:6; The Rf values of the raw materials and various products are 0.7 for 345 methyl ester, 0.6 for 35 methyl ester, and 0.3 for compound 3. The characterization data of the compound are as follows:
hydrogen nuclear magnetic spectrum ¹H NMR (400 MHz, CDCl₃) δ= 6.98 (s, 2H), 6.93 (d, *J* = 1.8 Hz, 3H), 6.80 (d, *J* = 1.6 Hz, 3H), 5.08 (s, 1H), 4.68 (s, 3H), 3.93 (d, *J* = 5.9 Hz, 8H), 2.82 (dd, *J* = 15.3, 7.7 Hz, 8H), 2.57 (t, *J =* 7.7 Hz, 8H), 2.19 (s, 9H), 1.43 (s, 19H), 1.39 (s, 28H).
carbon nuclear magnetic spectrum ¹³C NMR (101 MHz, CDCl₃) δ 172.64 , 172.65, 152.27, 151.19, 136.05, 136.06, 131.27 , 130.72, 128.13, 124.80, 124.81, 123.51, 62.35 , 41.66, 36.09, 34.44, 34.45, 30.80, 30.40, 30.41, 29.80 , 16.01.

High resolution mass spectrometry HRMS (EI TOF, M+K⁺): 1089.6063.

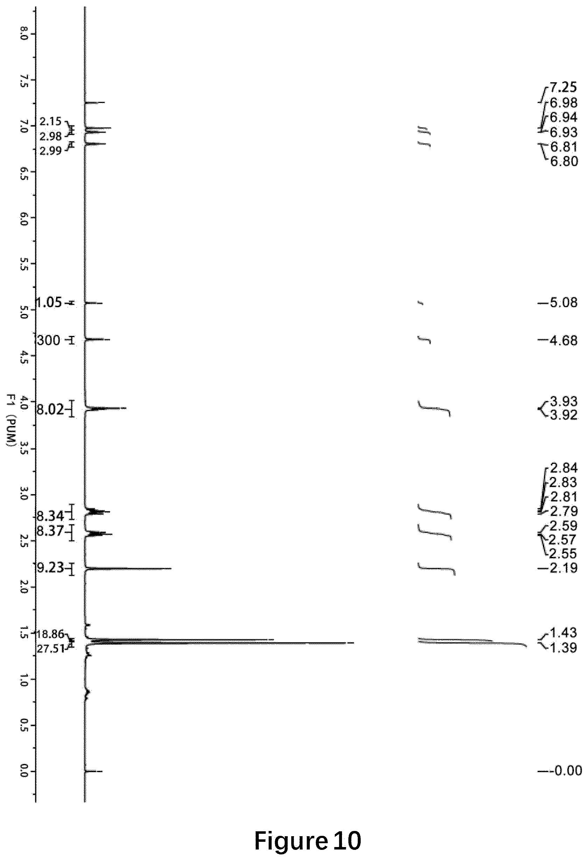

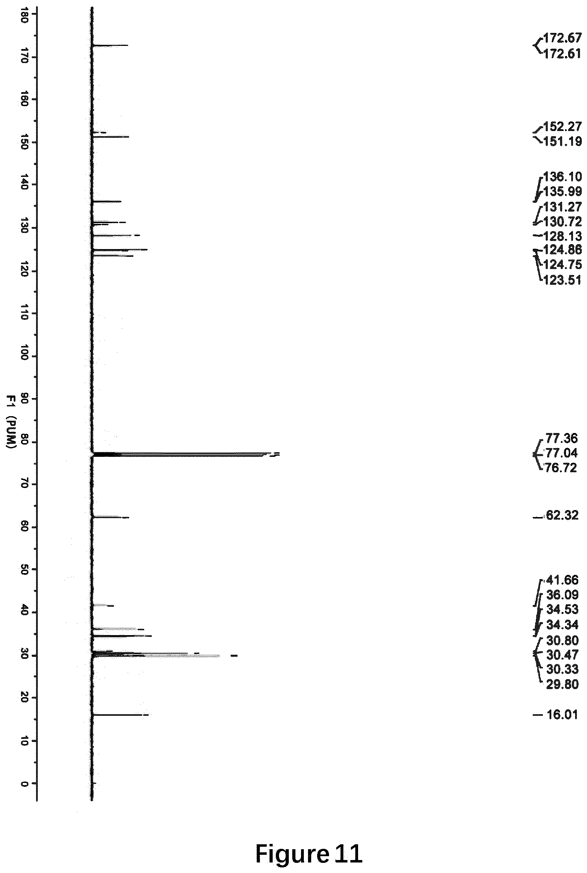

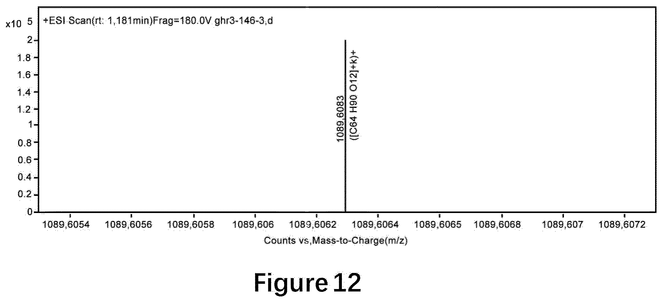

The infrared spectrum is shown in Figure 9, the hydrogen nuclear magnetic spectrum is shown in Figure 10, the carbon nuclear magnetic spectrum is shown in Figure 11, and the high-resolution mass spectrometry is shown in Figure 12.

### Characterization of application performance:

### TPU antioxidation performance:

Polyurethane elastomer (TPU) pellets were respectively prepared by the samples of compound 2 synthesized in Example 1, compound 1 synthesized in Example 3, compound 3 synthesized in Example 4, and commercially available antioxidant 1010 at a mass fraction of 2%. Test sheets **a1** (corresponding to compound 2), **a2** (corresponding to compound 1), **a3** (corresponding to compound 3), and **b** (corresponding to antioxidant 1010) were prepared through an elastomer injection machine, respectively. As a control, TPU pellets without antioxidant were prepared into a test sheet **c** using an elastomer injection machine;
All test sheets were subjected to aging test in a 90°C constant temperature bath, and then the mechanical properties of each elastomer test sheet were tested. Table 1 below shows the test results of each test sheet before and after the aging test. It can be seen from the results in the table that the polyurethane elastomer sheets added with compound 1, 2 and 3 samples have better anti-aging performance.

**Table 1**

| project | Aging time (day) | | | | | | | | | |
|---|---|---|---|---|---|---|---|---|---|---|
| | Test sheet a1 | | Test sheet a2 | | Test sheet a3 | | Test sheet b | | test piece c as a control | |
| | 0 | 20 | 0 | 20 | 0 | 20 | 0 | 20 | 0 | 20 |
| hardness | 92 | 91 | 92 | 90 | 92 | 91 | 92 | 90 | 92 | 90 |
| Tensile strength/MPa | 45 | 30 | 44 | 28 | 43 | 26 | 42 | 21 | 40 | 1 |
| Tear strength/MPa | 10.8 | 4.8 | 10.8 | 4.7 | 10.6 | 4.3 | 10.5 | 3.2 | 10.0 | 1.3 |
| 100% modulus strength/MPa | 0.74 | 0.45 | 0.74 | 0.43 | 0.73 | 0.41 | 0.73 | 0.3 | 0.72 | 0.10 |
| 300% modulus strength/MPa | 1.3 | 0.7 | 1.3 | 0.6 | 1.3 | 0.6 | 1.3 | 0.3 | 1.3 | 0 |
| Elongation/% | 540 | 250 | 530 | 230 | 530 | 220 | 520 | 150 | 505 | 50 |

### PP antioxidation performance:

The samples of compound 2 synthesized in Example 1, compound 1 synthesized in Example 3, compound 3 synthesized in Example 4, commercially available antioxidant Irganox 1010 in the market, and thiopropionate DSTDP were all applied to the stability effect experiment of polypropylene PP (homopolymer MI=2) according to the mass fraction specified in the table. After the materials were evenly mixed, they were put into a twin screw extruder for extrusion, with an extrusion temperature of 210-230°C. After cooling, drying, pelletizing, and sieving, a modified polypropylene is obtained.

The aging data of these modified polypropylenes in a Gill oven at 160°C were investigated, and the results were shown in Table 2 (note the basic formulation: PP contains 0.05% calcium stearate by weight).

As seen from the results in the table, when compounds 2, 1 and 3, and 1010 of the antioxidant samples are used alone as the main antioxidants, their stability to polypropylene is equivalent; when the main antioxidant is combined with the auxiliary antioxidant thiopropionate at the same mass, they can produce a synergistic stabilizing effect. Antioxidant compound 2 is obviously better than 1010, and has better anti-aging performance than the main antioxidant alone.

**Table 2**

| Antioxidant formulation | amount | 5% embrittlement time/h |
|---|---|---|
| No antioxidant | - | 5 |
| Irganox 1010 | 0.05% | 155 |
| Compound 2 | 0.05% | 180 |
| Compound 1 | 0.05% | 167 |
| Compound 3 | 0.05% | 170 |
| DSTDP | 0.15% | 50 |
| Irganox 1010/DSTDP | 0.05%/0.15% | 640 |
| Compound 2/DSTDP | 0.05%/0.15% | 1300 |
| Compound 1/DSTDP | 0.05%/0.15% | 950 |
| Compound 3/DSTDP | 0.05%/0.15% | 1020 |

### Characterization of yellowing resistance

As the concentration of NOx in the atmosphere continues to increase as a result of automobile and chemical exhaust emissions, the coloring effect of NOx gas produced on polymer products is becoming increasingly prominent. It is also important to investigate the coloring performance of antioxidant NOx. Comparing the samples of compounds 2, 1, and 3, as well as the commercially available antioxidant Irganox 1010 in the market, as a powder, when exposed to 3% nitrogen dioxide gas (NO2) and left at room temperature for 1.5 hours, it was observed that the antioxidant Irganox 1010 powder had completely turned yellow, while the powder of compounds 2, 1, and 3 were almost off-white with almost no change. It illustrates that the intramolecular complex hindered phenolic compound provided by the present invention has good yellowing resistance and is more resistant to NOx coloring.

## Claims

1. An intramolecular complex hindered phenolic compound, **characterized in that** the intramolecular complex hindered phenolic compound has a structure represented by formula I: 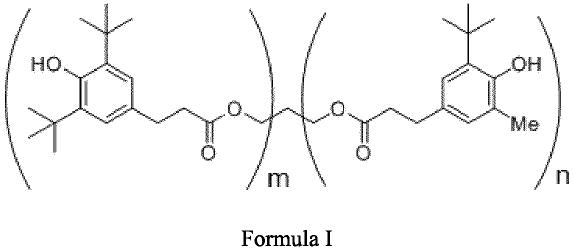 wherein, m is 1-3, n is 1-3, and m+n is 4.

2. The intramolecular complex hindered phenolic compound according to claim 1, **characterized in that** m is 1 and n is 3; or, m is 2 and n is 2; or, m is 3 and n is 1; and preferably, m is 2 and n is 2.

3. A method for preparing the intramolecular complex hindered phenolic compound according to claim 1 or 2, **characterized in that** the method comprises the following steps:
a first transesterification reaction is conducted between pentaerythritol and a first monomer to obtain an intermediate substitution product; a second transesterification reaction is conducted between the intermediate substitution product and a second monomer to obtain the intramolecular complex hindered phenolic compound; or,
a third transesterification reaction is conducted between pentaerythritol and a monomer mixture to obtain the intramolecular complex hindered phenolic compound; the monomer mixture includes the first monomer and the second monomer;
wherein, the first monomer and the second monomer are different, and are respectively selected from methyl 3-methyl-5-tert-butyl-4-hydroxyphenylpropionate or methyl 3,5-di-tert-butyl-4-hydroxyphenylpropionate; the substituent number of the first monomer in the intermediate substitution product corresponds to m or n in formula I; the molar ratio of the first monomer to the second monomer in the monomer mixture corresponds to the ratio of m to n in Formula I.

4. The method for preparing the intramolecular complex hindered phenolic compound according to claim 3, **characterized in that** the steps in the first transesterification reaction, the second transesterification reaction, and the third transesterification reaction are all carried out under the action of an ester exchange catalyst;
preferably, the transesterification catalyst is selected from one or more of alkyltin oxide, lithium amide, sodium methoxide, lithium methoxide, alkoxyaluminum, and zinc salt of an organic acid;
more preferably, the alkyltin oxide is selected from one or more of monobutyltin oxide and dioctyltin oxide.

5. The method for preparing the intramolecular complex hindered phenolic compound according to claim 4, **characterized in that** the first monomer is methyl 3-methyl-5-tert-butyl-4-hydroxyphenylpropionate, and the second monomer is methyl 3,5-di-tert-butyl-4-hydroxyphenylpropionate; preferably, the method comprises the following steps:
pentaerythritol, the first monomer, and the transesterification catalyst are mixed under an inert gas, and subjected to the first transesterification reaction to obtain a pre-reaction system comprising the intermediate substitution product;
the second monomer is added to the pre-reaction system and subjected to the second transesterification reaction to obtain the intramolecular complex hindered phenolic compound.

6. A polymer material antioxidant, **characterized in that** it comprises one or more of the intramolecular complex hindered phenolic compounds according to claim 1 or 2.

7. The polymer material antioxidant according to claim 6, **characterized in that** the polymer material antioxidant further comprises phosphite antioxidant and/or thioester antioxidant; preferably, the phosphite ester antioxidant is one or more of tris(2,4-di-tert-butylphenyl) phosphite, bis(2,4-di-tert-butylphenyl)pentaerythritol diphosphite, bis(2,4- dicumylphenyl)pentaerythritol diphosphite, triisodecyl phosphite, diisodecyl pentaerythritol diphosphite, tetraphenyldipropylene glycol diphosphite, diphenyl monoisodecyl phosphite, triphenyl phosphite, monophenyl diisodecyl phosphite, tris(nonylphenyl) phosphite; preferably, the thioester antioxidant is one or more of pentaerythritol tetra(3-decylthiopropionate), dioctadecanol thiodipropionate, didodecanol thiodipropionate, ditetradecanol thiodipropionate, and ditridecanol thiodipropionate.

8. A polymer material, comprising a body of the polymer material and an antioxidant, **characterized in that** the antioxidant is the polymer material antioxidant according to claim 6 or 7.

9. The polymer material according to claim 8, **characterized in that** the body of the polymer material is resin or rubber; the resin is preferably polyolefin, polyurethane, polyether, polyketone, polystyrene, polycarbonate, polyacrylate, polymethacrylate, polyacetal, halogen-containing polymer, polyamide, polyformaldehyde, polyphenylene ether, polyethylene terephthalate, polybutylene terephthalate, polyacrylonitrile, polybutadiene, polystyrene, HIPS, ABS, MBS, epoxy resin or acrylic resin crosslinked with epoxy resin; and the rubber is preferably natural rubber, styrene butadiene rubber, chloroprene rubber, cis-1,4-polybutadiene rubber, isoprene rubber, nitrile rubber, or thermoplastic TPU.

10. The polymer material according to claim 8, **characterized in that**:
the body of the polymer material is thermoplastic TPU, and the addition amount of the antioxidant is 0.01-3% by weight of the body of the polymer material; alternatively,
the polymer material is PP, and the addition amount of the antioxidant is 0.01-1% by weight of the body of the polymer material; preferably, when the polymer material is PP, the antioxidant comprises the intramolecular complex hindered phenolic compound according to claim 1 or 2 and antioxidant DSTDP, and more preferably, the weight ratio of the two is1:5-5:1.

## Patentansprüche

1. Intramolekularkomplex-gehinderte phenolische Verbindung, **dadurch gekennzeichnet, dass** die Intramolekularkomplex-gehinderte phenolische Verbindung eine Struktur dargestellt durch Formel I aufweist: 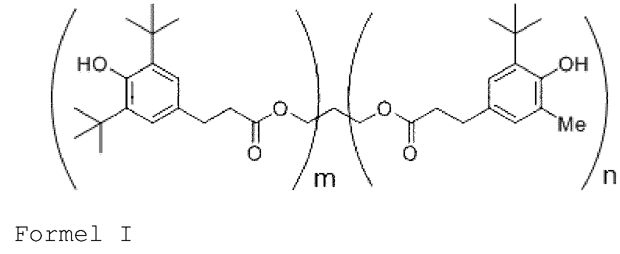 wobei m 1-3 ist, n 1-3 ist und m+n 4 ist.

2. Intramolekularkomplex-gehinderte phenolische Verbindung nach Anspruch 1, **dadurch gekennzeichnet, dass** m 1 ist und n 3 ist; oder m 2 ist und n 2 ist; oder m 3 ist und n 1 ist; und vorzugsweise m 2 ist und n 2 ist.

3. Verfahren zur Herstellung der Intramolekularkomplex-gehinderten phenolischen Verbindung nach Anspruch 1 oder 2, **dadurch gekennzeichnet, dass** das Verfahren die folgenden Schritte umfasst:
eine erste Umesterungsreaktion wird zwischen Pentaerythrit und einem ersten Monomer durchgeführt, um ein intermediäres Substitutionsprodukt zu erhalten; eine zweite Umesterungsreaktion wird zwischen dem intermediären Substitutionsprodukt und einem zweiten Monomer durchgeführt, um die Intramolekularkomplex- gehinderte phenolische Verbindung zu erhalten; oder
eine dritte Umesterungsreaktion wird zwischen Pentaerythrit und einem Monomergemisch durchgeführt, um die Intramolekularkomplex-gehinderte phenolische Verbindung zu erhalten; wobei das Monomergemisch das erste Monomer und das zweite Monomer enthält;
wobei das erste Monomer und das zweite Monomer verschieden sind und jeweils ausgewählt sind aus 3-Methyl-5-tert-butyl-4-
hydroxyphenylpropionsäuremethylester und 3,5-Di-tert- butyl-4-hydroxyphenylpropionsäuremethylester; die Substituentenzahl des ersten Monomers in dem intermediären Substitutionsprodukt m oder n in Formel I entspricht; das molare Verhältnis des ersten Monomers zu dem zweiten Monomer in dem Monomergemisch dem Verhältnis von m zu n in Formel I entspricht.

4. Verfahren zur Herstellung der Intramolekularkomplex-gehinderten phenolischen Verbindung nach Anspruch 3, **dadurch gekennzeichnet, dass** die Schritte der ersten Umesterungsreaktion, der zweiten Umesterungsreaktion und der dritten Umesterungsreaktion alle unter der Wirkung eines Esteraustauschkatalysators durchgeführt werden;
wobei der Umesterungskatalysator vorzugsweise ausgewählt ist aus einem oder mehreren von Alkylzinnoxid, Lithiumamid, Natriummethoxid, Lithiummethoxid, Alkoxyaluminium und Zinksalz einer organischen Säure;
bevorzugter das Alkylzinnoxid ausgewählt ist aus einem oder mehreren von Monobutylzinnoxid und Dioctylzinnoxid.

5. Verfahren zur Herstellung der Intramolekularkomplex-gehinderten phenolischen Verbindung nach Anspruch 4, **dadurch gekennzeichnet, dass** das erste Monomer 3-Methyl-5-tert-butyl-4- hydroxyphenylpropionsäuremethylester ist und das zweite Monomer 3,5-Di-tert-butyl-4- hydroxyphenylpropionsäuremethylester ist; wobei das Verfahren vorzugsweise die folgenden Schritte umfasst:
Pentaerythrit, das erste Monomer und der Umesterungskatalysator werden unter einem Inertgas gemischt und der ersten Umesterungsreaktion unterzogen, um ein Vorreaktionssystem zu erhalten, das das intermediäre Substitutionsprodukt umfasst;
das zweite Monomer wird zu dem Vorreaktionssystem zugegeben und der zweiten Umesterungsreaktion unterworfen, um die Intramolekularkomplex-gehinderte phenolische Verbindung zu erhalten.

6. Polymermaterial-Antioxidationsmittel, **dadurch gekennzeichnet, dass** es eine oder mehrere der Intramolekularkomplex-gehinderten phenolischen Verbindungen nach Anspruch 1 oder 2 enthält.

7. Polymermaterial-Antioxidationsmittel nach Anspruch 6, **dadurch gekennzeichnet, dass** das Polymermaterial-Antioxidationsmittel ferner Phosphit-Antioxidationsmittel und/oder Thioester-Antioxidationsmittel umfasst; wobei das Phosphitester-Antioxidationsmittel vorzugsweise eines oder mehrere von Tris(2,4-di-tert-butylphenyl)phosphit, Bis(2,4-di-tert-butylphenyl)pentaerythritdiphosphit, Bis(2,4-dicumylphenyl)pentaerythritdiphosphit, Triisodecylphosphit, Diisodecylpentaerythritdiphosphit, Tetraphenyldipropylenglycoldiphosphit, Diphenylmonoisodecylphosphit, Triphenylphosphit, Monophenyldiisodecylphosphit, Tris(nonylphenyl)phosphit ist; wobei das Thioester-Antioxidationsmittel vorzugsweise eines oder mehrere von Pentaerythrittetra(3-decylthiopropionat), Dioctadecanolthiodipropionat, Didodecanolthiodipropionat, Ditetradecanolthiodipropionat und Ditridecanolthiodipropionat ist.

8. Polymermaterial, umfassend einen Körper des Polymermaterials und ein Antioxidationsmittel, **dadurch gekennzeichnet, dass** das Antioxidationsmittel das Polymermaterial-Antioxidationsmittel nach Anspruch 6 oder 7 ist.

9. Polymermaterial nach Anspruch 8, **dadurch gekennzeichnet, dass** der Körper des Polymermaterials Harz oder Kautschuk ist; das Harz vorzugsweise Polyolefin, Polyurethan, Polyether, Polyketon, Polystyrol, Polycarbonat, Polyacrylat, Polymethacrylat, Polyacetal, halogenhaltiges Polymer, Polyamid, Polyformaldehyd, Polyphenylenether, Polyethylenterephthalat, Polybutylenterephthalat, Polyacrylnitril, Polybutadien, Polystyrol, HIPS, ABS, MBS, Epoxidharz oder mit Epoxidharz vernetztes Acrylharz ist; und der Kautschuk vorzugsweise Naturkautschuk, Styrolbutadienkautschuk, Chloroprenkautschuk, cis-1,4-Polybutadienkautschuk, Isoprenkautschuk, Nitrilkautschuk oder thermoplastisches TPU ist.

10. Polymermaterial nach Anspruch 8, **dadurch gekennzeichnet, dass**:
der Körper des Polymermaterials thermoplastisches TPU ist und die Zugabemenge des Antioxidationsmittels 0,01-3 Gew.-% des Körpers des Polymermaterials beträgt; alternativ
das Polymermaterial PP ist und die Zugabemenge des Antioxidationsmittels 0,01-1 Gew.-% des Körpers des Polymermaterials beträgt; vorzugsweise, wenn das Polymermaterial PP ist, das Antioxidationsmittel die Intramolekularkomplex-gehinderte phenolische Verbindung nach Anspruch 1 oder 2 und Antioxidationsmittel DSTDP umfasst und bevorzugter das Gewichtsverhältnis der beiden 1:5-5:1 beträgt.

## Revendications

1. Composé phénolique encombré de type complexe intramoléculaire, **caractérisé en ce que** le composé phénolique encombré de type complexe intramoléculaire présente une structure représentée par la formule I : 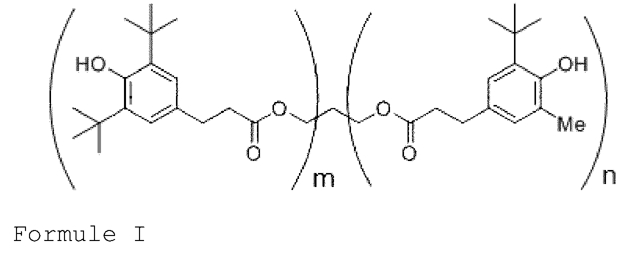 dans lequel, m représente 1 à 3, n représente 1 à 3, et m + n représente 4.

2. Composé phénolique encombré de type complexe intramoléculaire selon la revendication 1, **caractérisé en ce que** m représente 1 et n représente 3 ; ou, m représente 2 et n représente 2 ; ou, m représente 3 et n représente 1 ; et de préférence, m représente 2 et n représente 2.

3. Procédé de préparation d'un composé phénolique encombré de type complexe intramoléculaire selon la revendication 1 ou 2, **caractérisé en ce que** le procédé comprend les étapes suivantes :
une première réaction de transestérification est effectuée entre le pentaérythritol et un premier monomère pour obtenir un produit de substitution intermédiaire ;
une deuxième réaction de transestérification est effectuée entre le produit de substitution intermédiaire et un second monomère pour obtenir le composé phénolique encombré de type complexe intramoléculaire ; ou,
une troisième réaction de transestérification est effectuée entre le pentaérythritol et un mélange de monomères pour obtenir le composé phénolique encombré de type complexe intramoléculaire ; le mélange de monomères inclut le premier monomère et le second monomère ;
dans lequel, le premier monomère et le second monomère sont différents, et sont respectivement choisis parmi le 3-méthyl-5-tert-butyl-4-hydroxyphénylpropionate de méthyle ou le 3,5-di-tert-butyl-4-hydroxyphénylpropionate de méthyle ; le nombre de substituants du premier monomère dans le produit de substitution intermédiaire correspond à m ou n dans la formule I ; le rapport molaire du premier monomère au second monomère dans le mélange de monomères correspond au rapport de m à n dans la formule I.

4. Procédé de préparation du composé phénolique encombré de type complexe intramoléculaire selon la revendication 3, **caractérisé en ce que** les étapes de la première réaction de transestérification, de la deuxième réaction de transestérification et de la troisième réaction de transestérification sont toutes réalisées sous l'action d'un catalyseur d'échange d'ester ;
de préférence, le catalyseur de transestérification est choisi parmi un ou plusieurs parmi l'oxyde d'alkylétain, l'amidure de lithium, le méthoxyde de sodium, le méthoxyde de lithium, l'alcoxyaluminium, et le sel de zinc d'un acide organique ;
plus préférentiellement, l'oxyde d'alkylétain est choisi parmi un ou plusieurs parmi l'oxyde de monobutylétain et l'oxyde de dioctylétain.

5. Procédé de préparation du composé phénolique encombré de type complexe intramoléculaire selon la revendication 4, **caractérisé en ce que** le premier monomère est le 3-méthyl-5-tert-butyl-4-hydroxyphénylpropionate de méthyle, et le second monomère est le 3,5-di-tert-butyl-4-hydroxyphénylpropionate de méthyle ; de préférence, le procédé comprend les étapes suivantes :
le pentaérythritol, le premier monomère et le catalyseur de transestérification sont mélangés sous un gaz inerte, et soumis à la première réaction de transestérification pour obtenir un système de pré-réaction comprenant le produit de substitution intermédiaire ;
le second monomère est ajouté au système de pré-réaction et soumis à la seconde réaction de transestérification pour obtenir le composé phénolique encombré de type complexe intramoléculaire.

6. Antioxydant pour matériau polymère, **caractérisé en ce qu'**il comprend un ou plusieurs des composés phénoliques encombrés de type complexe intramoléculaire selon la revendication 1 ou 2.

7. Antioxydant pour matériau polymère selon la revendication 6, **caractérisé en ce que** l'antioxydant pour matériau polymère comprend en outre un antioxydant phosphite et/ou un antioxydant thioester ; de préférence, l'antioxydant ester de phosphite est un ou plusieurs parmi le tris(2,4-di-tert-butylphényl)phosphite, le bis(2,4-di-tert-butylphényl)pentaérythritol diphosphite, le bis(2,4-dicumylphényl)pentaérythritol diphosphite, le triisodécylphosphite, le diisodécylpentaérythritol diphosphite, le tétraphényldipropylène glycol diphosphite, le diphénylmonoisodécylphosphite, le triphénylphosphite, le monophényldiisodécylphosphite, le tris(nonylphényl)phosphite ; de préférence, l'antioxydant thioester est un ou plusieurs parmi le tétra(3-décylthiopropionate) de pentaérythritol, le thiodipropionate de dioctadécanol, le thiodipropionate de didodécanol, le thiodipropionate de ditétradécanol, et le thiodipropionate de ditridécanol.

8. Matériau polymère, comprenant un corps du matériau polymère et un antioxydant, **caractérisé en ce que** l'antioxydant est l'antioxydant du matériau polymère selon la revendication 6 ou 7.

9. Matériau polymère selon la revendication 8, **caractérisé en ce que** le corps du matériau polymère est une résine ou un caoutchouc ; la résine est de préférence une polyoléfine, un polyuréthane, un polyéther, une polycétone, un polystyrène, un polycarbonate, un polyacrylate, un polyméthacrylate, un polyacétal, un polymère halogéné, un polyamide, un polyformaldéhyde, un polyphénylène éther, un polyéthylène téréphtalate, un polybutylène téréphtalate, un polyacrylonitrile, un polybutadiène, un polystyrène, un HIPS, un ABS, un MBS, une résine époxy ou une résine acrylique réticulée avec une résine époxy ; et de préférence le caoutchouc est du caoutchouc naturel, du caoutchouc styrène butadiène, du caoutchouc chloroprène, du caoutchouc cis-1,4-polybutadiène, du caoutchouc isoprène, du caoutchouc nitrile, ou du TPU thermoplastique.

10. Matériau polymère selon la revendication 8, **caractérisé en ce que** :
le corps du matériau polymère est du TPU thermoplastique, et la quantité d'addition de l'antioxydant est de 0,01 à 3 % en poids par rapport au corps du matériau polymère ; en variante,
le matériau polymère est du PP, et la quantité d'addition de l'antioxydant est de 0,01 à 1 % en poids par rapport au corps du matériau polymère ; de préférence, lorsque le matériau polymère est PP, l'antioxydant comprend le composé phénolique encombré de type complexe intramoléculaire selon la revendication 1 ou 2 et l'antioxydant DSTDP, et plus préférentiellement, le rapport pondéral des deux est de 1:5 à 5:1.
